(19) Europäisches Patentamt / European Patent Office / Office européen des brevets

(11) **EP 3 877 016 B1**

(12) **EUROPÄISCHE PATENTSCHRIFT**

(45) Veröffentlichungstag und Bekanntmachung des Hinweises auf die Patenterteilung:
**04.05.2022 Patentblatt 2022/18**

(21) Anmeldenummer: **20775283.3**

(22) Anmeldetag: **18.09.2020**

(51) Internationale Patentklassifikation (IPC):
**A61M 1/34** (2006.01)  **A61M 1/36** (2006.01)

(52) Gemeinsame Patentklassifikation (CPC):
**A61M 1/342; A61M 1/3486; A61M 1/3672;**
A61M 2205/3334

(86) Internationale Anmeldenummer:
**PCT/EP2020/076133**

(87) Internationale Veröffentlichungsnummer:
**WO 2021/053160 (25.03.2021 Gazette 2021/12)**

(54) **VERFAHREN UND VORRICHTUNG ZUR STEUERUNG DER ANTIKOAGULATION BEI DER EXTRAKORPORALEN BLUTBEHANDLUNG**

METHOD AND APPARATUS FOR CONTROLLING ANTICOAGULATION DURING EXTRACORPOREAL BLOOD TREATMENT

PROCÉDÉ ET APPAREIL DE RÉGULATION D'ANTICOAGULATION DURANT LE TRAITEMENT EXTRACORPOREL DU SANG

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.09.2019 DE 102019125355**

(43) Veröffentlichungstag der Anmeldung:
**15.09.2021 Patentblatt 2021/37**

(73) Patentinhaber: **B. Braun Avitum AG**
**34212 Melsungen (DE)**

(72) Erfinder: **MANDRY, Peter**
**01326 Dresden (DE)**

(74) Vertreter: **Winter, Brandl - Partnerschaft mbB**
**Alois-Steinecker-Straße 22**
**85354 Freising (DE)**

(56) Entgegenhaltungen:
EP-B1- 0 705 845    US-A- 4 898 675
US-A- 5 421 812

**Beschreibung**

Technisches Gebiet

Die vorliegende Erfindung bezieht sich auf eine Vorrichtung zur Steuerung der Antikoagulation während einer extrakorporalen Blutbehandlung.

Hintergrund der Erfindung

Eine Sepsis ist eine der schwersten Komplikationen akuter Infektionen und Infektionskrankheiten und zählt zu einer der häufigsten und kostenintensivsten Erkrankungen im stationären Sektor. Dadurch stellt die Sepsis eine erhebliche Herausforderung für Gesundheitssysteme weltweit dar. Kostenfaktoren, die mit der Behandlung von Sepsis-Patienten verbunden sind, sind unter anderem die häufigen Wiedereinweisungen und die in vielen Fällen langanhaltenden Krankheitsfolgen wie z.B. eine Langzeitbeatmung und Dialysepflichtigkeit, die eine Sepsis-Erkrankung oftmals mit sich bringt. Nicht zu vernachlässigen sind aber auch die Kosten, die mit einer auftretenden langfristigen Arbeitsunfähigkeit und einer damit verbundenen Frühverrentung einhergehen. So steht die Sepsis in den USA mit jährlichen Kosten in Höhe von 24 Milliarden US-Dollar an Platz eins der Krankenhausbehandlungskosten. Im Vergleich dazu wurden die direkten Behandlungskosten einer Sepsis 2013 in Deutschland im ambulanten und stationären Bereich auf 7,5 Milliarden Euro geschätzt.

Der lebensbedrohliche Zustand einer Sepsis entsteht, wenn die körpereigenen Abwehrreaktionen eine Infektion, die meist durch Bakterien, aber potentiell auch durch Viren, Pilze oder Parasiten ausgelöst wird, und deren Folgen nicht mehr lokal begrenzen können. Folglich kommt es zu einer überschießenden Abwehrreaktion des Körpers, die zu einer Schädigung der eigenen Gewebe und Organe führt. Wird eine Sepsis nicht zeitnah diagnostiziert und behandelt, kann sich der Zustand des Patienten innerhalb kürzester Zeit dramatisch verschlechtern und zu einem multiplen Organversagen z.B. des Herzens, der Lunge, der Leber und Nieren und/oder einem septischen Kreislaufschock führen und tödlich enden.

Eine Schlüsselrolle bei der überschießenden Abwehrreaktion des Körpers spielen sogenannte Lipopolysaccharide (LPS), die zur Gruppe der Endotoxine gehören. LPS sind relativ thermostabile Verbindungen, die aus einem hydrophilen Polysaccharid- und einem lipophilen Lipidanteil aufgebaut sind, und einen Hauptbestandteil der äußeren Zellwand gramnegativer Bakterien wie Salmonellen, Escherichia coli oder Legionellen darstellen. Bereits intravenöse Dosen von 1 ng/kg Körpergewicht pro Stunde können Entzündungsreaktionen beim Menschen verursachen, weshalb LPS als extrem toxisch gelten. LPS werden während der Zellteilung proliferierender gramnegativer Bakterien oder bei der Lyse/Zerstörung dieser Bakterien durch Antibiotika oder das menschliche Komplementsystem in die Blutbahn freigesetzt. Das menschliche Immunsystem erkennt diese Endotoxine und setzt infolgedessen eine Vielzahl von Abwehr- und Entzündungsreaktionen in Gang, die zu einer Überproduktion von regulatorischen Proteinen, sogenannten Zytokinen, führen. Durch eine derartige systemische Zytokin-Überproduktion kann es wiederum zur Schädigung von Blutgefäßen und einem septischen Schock, begleitet von einer disseminierten intravasalen Gerinnung und einem multiplen Organversagen kommen.

Die Entfernung von LPS aus dem Blut betroffener Patienten stellt daher einen vielversprechenden Ansatz dar, um die eingeleiteten Abwehr- und Entzündungsreaktionen schnell einzudämmen und die Produktion von Zytokinen zu reduzieren. Wie Falkenhagen et al. (Int J Artif Organs 2014; 37 (3): 222-232) im Rahmen der Prüfung der Wirksamkeit verschiedener kommerziell erhältlicher Endotoxinadsorber zeigen konnten, ermöglichen lediglich Adsorber mit Diethylaminoethylcellulose (DEAE)-modifizierten Oberflächen, die jedoch bisher nicht als klinisch einsetzbar gelten, eine effektive Entfernung von LPS aus Blutserum. Gemäß der durchgeführten Studie handelt es sich bei einem derartigen Adsorber um einen Anionenaustauscher, der in der Lage ist, Moleküle mit negativer Ladung zu binden. Dazu verfügt der Anionenaustauscher über kationisches Material, in diesem Fall über eine DEAE-modifizierte Oberfläche, welches kovalent an eine feste unlösliche Matrix gebunden ist, und über daran ionisch gebundene neutralisierende Anionen, die durch das Binden anderer Anionen ausgetauscht werden.

Bei einer Blutbehandlung im Rahmen extrakorporaler Therapien werden dem Blut des jeweiligen Patienten in einem extrakorporalen Kreislauf sogenannte Antikoagulantien/Gerinnungshemmer wie beispielsweise Heparin zugemischt, um eine Blutgerinnung des zu behandelnden Blutes zu hemmen. Von besonderer Bedeutung bei der Blutbehandlung von Sepsis-Patienten bzw. Patienten mit septischen Schock mittels extrakorporaler Therapien ist dabei die Konzentration des Antikoagulans, da eine zu geringe Konzentration zur Gerinnung des Blutes und damit zu einem Therapiestopp führt, während eine zu hohe Konzentration des Antikoagulans bei der Rückführung des behandelten Blutes in den Körper des Patienten zu inneren Blutungen und sogar bis hin zum Tod führen kann. Aufgrund dessen ist eine gleichbleibende Konzentration des gerinnungshemmenden Mittels essentiell für eine erfolgreiche extrakorporale Blutbehandlung von Sepsis-Patienten.

Heparin ist ein bevorzugtes Antikoagulans, das besonders in der Intensivmedizin zur Versorgung einer Sepsis Anwendung findet. Heparine sind variabel veresterte Glycosaminoglycane mit einer Vielzahl an negativen Ladungen, die ab einer Kettenlänge von fünf Monosacchariden ihre gerinnungshemmende Wirkung entfalten. Durch Bindung an den Proteaseinhibitor Antithrombin III bewirkt Heparin, dass die Bindung von Antithrombin III an Gerinnungsfaktoren etwa tausendfach schneller abläuft und infolgedessen die Blutgerinnung gehemmt wird. Aufgrund seiner negativen Gesamtladung eignet sich Heparin allerdings nicht für den Einsatz im Rahmen einer extrakorporalen Blutbehandlung zur Entfernung von LPS an einem mit DEAE beschichteten Anionenaustauscher, da der Anionenaustauscher neben LPS auch Heparin bindet. Ein Entfernen von Heparin hat jedoch wie vorstehend beschrieben die Gerinnung des Patientenblutes und damit den Abbruch der Therapie zur Folge. Außerdem werden Sepsis-Patienten zur Verhinderung einer Thrombose mit geringen Dosen Heparin behandelt. Auch dieses Heparin würde durch einen mit DEAE beschichteten Anionenaustauscher entfernt werden. Somit gibt es Bedarf an Therapiemöglichkeiten von Sepsis-Patienten bzw. Patienten mit septischen Schock, die eine effektive Entfernung von LPS ermöglichen, ohne dass Nebenwirkungen oder Risiken für den Patienten entstehen.

Stand der Technik

Aus dem Stand der Technik ist das sogenannte HELP-Verfahren (Heparininduzierte extrakorporale LDL-Präzipitation) bekannt, welches unter anderem in der DE 31 35 814 A1 offenbart ist. Das HELP-Verfahren wird bisher zur chronischen Behandlung von Patienten mit einer angeborenen Fettstoffwechselstörung, der schweren primären Hypercholesterinämie, eingesetzt. Dabei wird das zuvor separierte Plasma mit Heparin und Azetatpuffer versetzt, wodurch der pH-Wert des Plasmas sinkt. Das unter diesen Bedingungen erzeugte Präzipitat aus LDL, Fibrinogen und Heparin wird anschließend herausgefiltert und aus dem Plasmakreislauf entfernt. Das danach noch vorhandene überschüssige Fällungsreagenz Heparin wird in einem weiteren Schritt an einem DEAE-Cellulose-Anionenaustauscher selektiv adsorbiert und entfernt. Durch die Entfernung der Pufferlösung mittels einer Dialyse und Ultrafiltration wird im Anschluss der physiologische pH-Wert wiederhergestellt, sodass das behandelte Plasma wieder mit den Blutzellen vermischt und letztlich dem Patienten zurückgeführt werden kann.

Um das Gerinnen des Bluts zu verhindern und die Entfernung des Heparins durch den Anionenaustauscher auszugleichen, wird dem Blut des Patienten im Rahmen des in dem in DE 31 35 814 A1 offenbarten HELP-Verfahrens ein höherer Bolus an Heparin beigemischt. Aufgrund der vorstehend erwähnten überschießenden Abwehrreaktion des Körpers ist die Blutgerinnung bei Sepsis-Patienten bzw. Patienten mit septischen Schock jedoch gestört und instabil, weshalb eine konstante Heparinkonzentration essentiell für solche Patienten ist. Aus diesem Grund ist eine einfache Überdosierung des Heparins wie bei Patienten, die mit Hilfe des HELP-Verfahrens behandelt werden, im Falle von Sepsis-Patienten bzw. Patienten mit septischen Schock nicht möglich und sogar lebensgefährlich.

Auch die EP 0 705 845 B1 offenbart ein Verfahren, welches weitgehend analog zu dem vorstehend beschriebenen HELP-Verfahren abläuft. Bei diesem wird die simultane Entfernung von Tumor-Nekrose-Faktor alpha (TNFa) und bakteriellen LPS aus einer wässrigen Flüssigkeit beschrieben. Dazu wird auch hier dem Blut bzw. Plasma nach gegebenenfalls nötiger Entfernung von korpuskulären Blutbestandteilen ein höherer Bolus an Heparin beigemischt, wodurch eine Fällung von TNFa bedingt wird. Nach Abfiltrieren oder Abzentrifugieren des TNFa-Präzipitats wird das überschüssige Heparin durch Bindung an einen Anionenaustauscher, welcher mit DEAE-Cellulose modifiziert ist, entfernt. Durch dessen negative Ladung wird jedoch neben dem Heparin auch LPS effektiv durch Bindung an den Anionenaustauscher entfernt. Analog zum HELP-Verfahren wird der physiologische pH-Wert des Bluts oder Plasmas auch in dem in EP 0 705 845 B1 offenbarten Verfahren im Anschluss durch Ultrafiltration und/oder einen zusätzlichen Dialyseschritt regeneriert.

Die Simulation des Verfahrens hat gezeigt, dass ohne eine Nachdosierung über 98% des Heparins aus dem Plasma durch Bindung an den Anionenaustauscher eliminiert werden. Ein Entfernen des Heparins in einem derartigen Umfang hat jedoch wie bereits vorstehend angedeutet den Nachteil, dass sich dessen blutverdünnende Wirkung minimiert bzw. wegfällt und das Blut gerinnt. Eine zu geringe Heparinkonzentration führt jedoch wie vorstehend beschrieben zu einem Abbruch der extrakorporalen Blutbehandlung, da eine darauf basierende Fortführung der Therapie das Risiko erhöht, dass mögliche durch die Gerinnung entstehende Blutgerinnsel zum Patienten zurückgeführt werden und im Körper des Patienten ggf. Thromben verursachen, die zu gravierenden Komplikationen wie einem Herzinfarkt oder Schlaganfall führen können. Umgekehrt, kann eine zu hohe Nachdosierung von Heparin zu inneren Blutungen führen. Eine ausreichende Sicherheit des Patienten ist daher bei der Anwendung des vorstehenden Verfahrens in der Praxis nicht gegeben.

Der Einsatz anderer Antikoagulantien in derartigen Verfahren ist wiederum oftmals weniger gut klinisch getestet oder mit hohen Kosten verbunden, weshalb sie in der Praxis weniger Anwendung finden.

US 2016 / 0038666 A1 offenbart Systeme und Verfahren zur Durchführung einer Nierenersatztherapie mit oder unter Verwendung eines Dialysators, Kontrollkomponenten, Sorptionsmittelpatrone und Flüssigkeitsreservoirs, die so konfiguriert sind, dass sie ein Gewicht und eine Größe aufweisen, die zum Tragen oder Tragen durch eine behandlungsbedürftige Person geeignet sind. Das System zur Durchführung einer Nierenersatztherapie verfügt über einen kontrollierten Compliance-Dialysekreislauf, bei dem eine Kontrollpumpe die bidirektionale Bewegung von Flüssigkeit über eine Dialysemembran steuert. Der Dialysekreislauf und ein extrakorporaler Kreislauf zum Zirkulieren von Blut stehen über die Dialysemembran in flüssiger Verbindung. Der Flüssigkeitsfluss, der sich zwischen dem extrakorporalen Kreislauf und dem Dialysekreislauf bewegt, wird durch die Geschwindigkeit, mit der die Steuerpumpe arbeitet, so modifiziert, dass eine Geschwindigkeit der Ultrafiltration und des konvektiven Spiels gesteuert werden kann. Das System sieht die Überwachung der Einlass- und Auslassleitfähigkeit der Sorptionsmittelpatrone vor, um eine Einrichtung zum Quantifizieren oder Überwachen der Entfernung von Harnstoff durch die Sorptionsmittelpatrone bereitzustellen.

Somit betrifft US 2016 / 0038666 A1 das Entfernen von positiv-gesamtgeladenen Kationen mittels eines Kationenaustauschers, welches sich auf Dialyse-Patienten mit chronischem Nierenversagen richtet. Darin ist offenbart, dass zur Dialyse zwecks der Abreicherung von Harnstoff bzw. Urea in einem ersten Schritt der Harnstoff mittels des Enzyms Urease in positivgeladene Ammoniumionen (NH4)+ und Kohlenstoffdioxid getrennt werden muss. In einem dieser enzymatischen Aufspaltung nachfolgenden Schritt werden die Ammoniumionen (NH4)+ mittels einer Ionenaustauscher-Stufe dem Blut entzogen, wozu ein Kationenaustauscher verwendet werden muss.

AT 509 192 A4 offenbart eine Alternative zu der Verwendung von Anionenaustauschern auf Grundlage des anderen Prinzips der Sorption, nämlich ein Sorptionsmittel zum Entfernen von Endotoxinen aus einer biologischen Flüssigkeit, umfassend einen wasserunlöslichen, porösen Träger mit einer neutralen, hydrophoben Oberfläche. Dabei weist die Oberfläche des Trägers eine adsorptive Beschichtung aus Polymyxin und Albumin auf, wobei Polymyxin und Albumin nichtkovalent mit der Oberfläche des Trägers verbunden sind. Somit befasst sich die AT 509 192 A4 im Sinne der Patientensicherheit mit der Immobilisierung des als neurotoxisch und nephrotoxisch vorbeschriebenen Polymyxins.

Offenbarung der Erfindung

Aufgabe der vorliegenden Erfindung ist es daher, eine Vorrichtung bereitzustellen, um eine effektive Eliminierung von LPS unter Verwendung eines Anionenaustauschers und dem zeitgleichen Einsatz einer Anzahl von/mindestens einer biologisch und/oder pharmakologisch aktiven Substanz(en) mit negativer Ladung, insbesondere von Heparin als Antikoagulans, im Rahmen einer extrakorporalen Blutreinigung zur Behandlung von Sepsis-Patienten bzw. Patienten mit septischen Schock zu ermöglichen. In diesem Zusammenhang besteht eine weitere Aufgabe der Erfindung darin die Zugabe von der Anzahl von biologisch und/oder pharmakologisch aktiven Substanzen mit negativer Ladung, insbesondere von Heparin, während der Therapie derart zu steuern, dass die Konzentration der Anzahl von biologisch und/oder pharmakologisch aktiven Substanzen mit negativer Ladung im extrakorporalen Kreislauf trotz der Adsorption an dem Anionenaustauscher konstant bleibt.

Eine weitere Aufgabe der vorliegenden Erfindung ist es in diesem Zusammenhang auch, die Sicherheit von Sepsis-Patienten bzw. Patienten mit septischen Schock während der extrakorporalen Blutreinigung zu erhöhen, indem durch Blutgerinnsel oder Blutungen erzeugte unerwünschte Nebenwirkungen und eine Unterbrechung der Therapie vermieden werden, und die Therapierung von Sepsis generell zu verbessern.

Diese Aufgabe wird durch eine (Antikoagulationssteuerung-)Vorrichtung mit den Merkmalen des Anspruchs 1 gelöst. Vorteilhafte Weiterbildungen der Offenbarung sind jeweils Gegenstände der Unteransprüche.

Die vorliegende Offenbarung umfasst eine Antikoagulationssteuerungsvorrichtung zur Anwendung während einer extrakorporalen Blutbehandlung mit einem Mittel, insbesondere einer Blutpumpe, zum Fördern von Blut in einem ersten Leitungsabschnitt; einem Mittel, insbesondere einer Heparinpumpe, zum Zuführen von Heparin zu dem Blut in den ersten Leitungsabschnitt; einem Mittel, insbesondere einem Plasmaseparator, zum Separieren des mit Heparin versetzten Blutes in korpuskuläre Blutbestandteile und Blutplasma; einem Mittel, insbesondere einer Plasmapumpe, zum Fördern des separierten Blutplasmas in einem zweiten Leitungsabschnitt über einen Anionenaustauscher zur Adsorption von Lipopolysacchariden; und einem Mittel zum Zusammenführen des behandelten Blutplasmas und der korpuskulären Blutbestandteile in einem dritten Leitungsabschnitt. Die Antikoagulationssteuerungsvorrichtung ist dabei gekennzeichnet durch ein Mittel zum Bestimmen eines ersten Volumenstroms des Blutplasmaanteils in dem ersten Leitungsabschnitt vor der Zuführung des Heparins, insbesondere an der Blutpumpe; ein Mittel zum Bestimmen eines zweiten Volumenstroms des Blutplasmas in dem zweiten Leitungsabschnitt stromaufwärts oder stromabwärts des Anionenaustauschers, insbesondere an der Plasmapumpe; und eine Steuereinheit, die dazu ausgelegt ist eine dem Blut vor dem Separieren zugeführte Heparinmenge auf der Grundlage des Verhältnisses von dem ersten Volumenstrom und dem zweiten Volumenstrom derart zu steuern, dass eine Heparinkonzentration in dem dritten Leitungsabschnitt nach dem Zusammenführen des gereinigten Blutplasmas und der korpuskulären Blutbestandteile einen vorbestimmten, insbesondere konstanten, Sollwert annimmt. Ein Mittel zur Bestimmung des zweiten Volumenstroms kann dabei beispielsweise die Förderrate der Pumpe oder ein Sensor wie ein Durchflusssensor oder Drucksensor sein. Die Antikoagulationssteuerungsvorrichtung ist dabei generell derart ausgelegt, dass eine Anzahl biologisch und/oder pharmakologisch aktiver Substanzen mit negativer Gesamtladung auf die gleiche Art und Weise wie Heparin zugeführt werden können.

Demgemäß umfasst die vorliegende Offenbarung im Allgemeinen eine Antikoagulationssteuerungsvorrichtung zur Anwendung während einer extrakorporalen Blutbehandlung mit: einem Mittel, insbesondere einer Blutpumpe, zum Fördern von Blut in einem ersten Leitungsabschnitt; einem Mittel, insbesondere einer Pumpe, zum Zuführen einer Anzahl von biologisch und/oder pharmakologisch aktiven Substanzen mit negativer Gesamtladung, insbesondere von Heparin, zu dem Blut in den ersten Leitungsabschnitt; einem Mittel, insbesondere einem Plasmaseparator, zum Separieren des mit der Anzahl von biologisch und/oder pharmakologisch aktiven Substanzen mit negativer Gesamtladung versetzten Blutes in korpuskuläre Blutbestandteile und Blutplasma; einem Mittel, insbesondere einer Plasmapumpe, zum Fördern des separierten Blutplasmas in einem zweiten Leitungsabschnitt über einen Anionenaustauscher zur Adsorption von Lipopolysacchariden; und einem Mittel zum Zusammenführen des behandelten Blutplasmas und der korpuskulären Blutbestandteile in einem dritten Leitungsabschnitt. Die Antikoagulationssteuerungsvorrichtung ist dabei gekennzeichnet durch ein Mittel zum Bestimmen eines ersten Volumenstroms des Blutplasmaanteils in dem ersten Leitungsabschnitt vor der Zuführung der Anzahl von biologisch und/oder pharmakologisch aktiven Substanzen mit negativer Gesamtladung, insbesondere an der Blutpumpe; ein Mittel zum Bestimmen eines zweiten Volumenstroms des Blutplasmas in dem zweiten Leitungsabschnitt stromaufwärts oder stromabwärts des Anionenaustauschers, insbesondere an der Plasmapumpe; und eine Steuereinheit, die dazu ausgelegt ist eine dem Blut vor dem Separieren zugeführte Menge der Anzahl von biologisch und/oder pharmakologisch aktiven Substanzen mit negativer Gesamtladung auf der Grundlage des Verhältnisses von dem ersten Volumenstrom und dem zweiten Volumenstrom derart zu steuern, dass eine Konzentration der Anzahl von biologisch und/oder pharmakologisch aktiven Substanzen mit negativer Gesamtladung in dem dritten Leitungsabschnitt nach dem Zusammenführen des behandelten Blutplasmas und der korpuskulären Blutbestandteile einen vorbestimmten, insbesondere konstanten, Sollwert annimmt.

Dabei wird durch die vorgeschlagene Offenbarung das grundsätzliche Dilemma einer mangelnden Selektivität von Anionenaustauschern hinsichtlich der gleichermaßen negativ-gesamtbeladenen Substanzen bzw. Stoffe bzw. Protagonisten bzw. Moleküle, nämlich einerseits von aus dem Blut abzutrennenden Endotoxinen, insbesondere den Lipopolysacchariden, als für einen (Sepsis-)Patienten hochgiftigen Substanzen versus andererseits von Gerinnungshemmern wie beispielsweise Heparin als für den (Sepsis-) Patienten überlebenswichtigen Substanzen, überwunden.

Da der Anionenaustauscher aufgrund seiner Funktionsweise neben Heparin auch andere Plasmaproteine mit negativer Gesamtladung, wie beispielsweise Gerinnungsfaktoren, entfernt, kann mit Hilfe des der Offenbarung zugrundeliegenden mathematischen Modells generell eine Anzahl/mindestens eine biologisch und/oder pharmakologisch aktive(r) Substanz(en) mit negativer Gesamtladung, zugeführt werden. Im Folgenden wird Heparin synonym für alle diese biologisch und/oder pharmakologisch aktiven Substanzen verwendet.

In anderen Worten wird, gemäß dem vorstehend beschriebenen Aspekt, durch eine Blutpumpe angesaugtes Blut an einer Vorrichtung zur extrakorporalen Behandlung zuerst mit Heparin, welches über eine Pumpe, insbesondere über eine Heparinpumpe, zugeführt wird, versetzt und im Anschluss daran in einem Plasmaseparator in korpuskuläre Bestandteile und Blutplasma separiert. Während die korpuskulären Bestandteile mit einem Teil des Blutplasmas in dem Plasmaseparator verbleiben, wird ein anderer Teil des Blutplasmas über einen Anionenaustauscher geleitet, an dem das in dem Blutplasma vorhandene LPS adsorbiert und folglich entfernt wird. Da neben dem LPS auch Heparin, aufgrund seiner negativen Ladung, an dem Anionenaustauscher adsorbiert wird, wird dem Blut vor der Auftrennung über die Heparinpumpe so viel Heparin zugegeben, dass die Heparinkonzentration nach dem Zusammenführen des behandelten Blutplasmas und der korpuskulären Bestandteile einen vorbestimmten konstanten Sollwert annimmt. Um jene Konstanz der Heparinkonzentration zu gewährleisten, wird eine über die Heparinpumpe zuzuführende Heparinmenge derart eingestellt, dass sie auf der Grundlage eines Volumenstroms des Blutplasmaanteils an der Blutpumpe vor der Zuführung des Heparins und eines Volumenstroms des Blutplasmas an der Plasmapumpe stromaufwärts oder stromabwärts des Anionenaustauschers basiert. Auf diese Weise wird der Einsatz eines Anionenaustauschers bei gleichzeitiger Verwendung von Heparin als Antikoagulans im Rahmen einer extrakorporalen Blutbehandlung, insbesondere für die Blutbehandlung von Sepsis, möglich.

Dabei zeigt sich als ein Vorteil der Offenbarung, dass es somit gelingen kann, also unter Inkaufnahme bzw. Umgehung der mangelnden Selektivität als technischem Nachteil bzw. überwindender Hürde von grundsätzlicher Natur, das technische Gesamtziel einerseits einer Entfernung von hochtoxischen Endotoxinen bei andererseits Vermeidung von Blutkoagulation mittels effektiver Unterdrückung der Blutgerinnungskaskade unter Einsatz von Blutgerinnungshemmern, insbesondere Heparin, mit einer konstanten Sollwert-Konzentration dennoch zu erreichen.

Weiter vorteilhaft ermöglicht die vorliegend offenbarte Vorrichtung eine erfolgreiche extrakorporale Blutbehandlung bereits unterhalb eines vollständigen Entzugs von Endotoxinen. Insoweit wirkt sich bereits eine teilweise Entfernung der Endotoxine bei einem Durchlauf zur Blutwäsche positiv aus.

Gemäß der Offenbarung kann die Antikoagulationssteuerungsvorrichtung ferner ein Mittel zum Bestimmen eines Istwerts der Konzentration der Anzahl von biologisch und/oder pharmakologisch aktiven Substanzen mit negativer Gesamtladung in dem dritten Leitungsabschnitt und ein Mittel zum Regeln der Konzentration der Anzahl von biologisch und/oder pharmakologisch aktiven Substanzen mit negativer Gesamtladung in dem dritten Leitungsabschnitt auf der Grundlage des bestimmten Istwerts, dem Verhältnis von dem ersten Volumenstrom und dem zweiten Volumenstrom und der zugeführten Menge der Anzahl von biologisch und/oder pharmakologisch aktiven Substanzen mit negativer Gesamtladung im ersten Leitungsabschnitt aufweisen.

Dabei kann das Mittel zum Regeln der Konzentration der Anzahl von biologisch und/oder pharmakologisch aktiven Substanzen mit negativer Gesamtladung in dem dritten Leitungsabschnitt ein Konzentrationsregelungsmittel bzw. ein Regler sein. Insbesondere dient dabei das Konzentrationsregelungsmittel bzw. der Regler einer, quasi dynamischen bzw. automatischen, Regelung der Heparin-Konzentration als SollWert / Führungsgröße auf Basis eines gemessenen Ist-Wertes der Heparin-Konzentration als Regelgröße, welcher eine, quasi einmalige, Sollwert-Einstellung bzw. -Steuerung der Heparin-Konzentration zugrundeliegt. Dies dient der, insbesondere dynamischen und/oder in Echtzeit gemonitorten, Überwachung der Ist-Heparin-Konzentration in dem den (Sepsis-) Patienten zurückgeleiteten Blut als Regelgröße.

Eine derartige Regelung ermöglicht eine kontinuierliche automatische Anpassung der Konzentration der Anzahl von biologisch und/oder pharmakologisch aktiven Substanzen mit negativer Gesamtladung in dem dritten Leitungsabschnitt unter Einbeziehung eventueller Störgrößen. So wird auch auf diese Weise eine konstante Konzentration der Anzahl von biologisch und/oder pharmakologisch aktiven Substanzen mit negativer Gesamtladung, insbesondere von Heparin, in dem dritten Leitungsabschnitt gewährleistet, was wie bereits vorstehend erläutert von essentieller Bedeutung bei der Blutbehandlung von Sepsis-Patienten bzw. Patienten mit septischen Schock ist.

Die Antikoagulationssteuerungsvorrichtung kann ferner ein Mittel zum Eingeben eines Hämatokrit-Werts des Blutes in dem ersten Leitungsabschnitt und ein Mittel zum Bestimmen des Volumenstroms des Blutplasmaanteils in dem ersten Leitungsabschnitt auf der Grundlage der Förderrate der Blutpumpe und des eingegebenen Hämatokrit-Werts aufweisen. Der zuvor bestimmte Hämatokrit-Wert des jeweiligen Patienten kann in diesem Zusammenhang zum Beispiel über eine an der Antikoagulationssteuerungsvorrichtung vorgesehene Benutzeroberfläche eingegeben werden. Die selbige Benutzeroberfläche kann auch als Mittel zum Eingeben der Förderrate der Plasmapumpe und der Blutpumpe, als Mittel zum Eingeben einer gewünschten Heparinkonzentration in dem dritten Leitungsabschnitt fungieren, die letztlich zu dem Patienten fließen soll. Die Förderrate der Blutpumpe kann wie im Fall der Plasmapumpe beispielsweise durch Sensoren wie Durchflusssensoren oder Drucksensoren bestimmt werden.

Um den vorstehend beschriebenen Volumenstrom des Blutplasmaanteils in dem ersten Leitungsabschnitt vor der Zuführung des Heparins bestimmen zu können, kann vorzugsweise die Antikoagulationssteuerungsvorrichtung ein Mittel zum Bestimmen eines Hämatokrit-Werts des Blutes in dem ersten Leitungsabschnitt, ein Mittel zum Bestimmen eines Volumenstroms des Blutes in dem ersten Leitungsabschnitt, insbesondere zum Bestimmen einer Förderrate der Blutpumpe, und ein Mittel zum Bestimmen des Volumenstroms des Blutplasmaanteils in dem ersten Leitungsabschnitt auf der Grundlage des bestimmten Volumenstroms des Blutes in dem ersten Leitungsabschnitt und des bestimmten Hämatokrit-Werts aufweisen.

Durch die Bestimmung des Hämatokrit-Werts, welcher den prozentualen Anteil der Zellbestandteile am Gesamtvolumen des Blutes widerspiegelt und patientenabhängig ist, und die Bestimmung des Volumenstroms des Blutes in dem ersten Leitungsabschnitt bzw. der Förderrate der Blutpumpe kann ein direkter Rückschluss auf den Volumenstrom des Blutplasmaanteils in dem ersten Leitungsabschnitt gezogen werden, der schließlich für die Berechnung der dem Blut in dem ersten Leitungsabschnitt zuzuführenden Heparinmenge benötigt wird.

Vorzugsweise kann die Antikoagulationssteuerungsvorrichtung ein Mittel zum Einstellen des zweiten Volumenstroms des durch den Anionenaustauscher strömenden Blutplasmas, insbesondere die Plasmapumpe und/oder an der Plasmapumpe, aufweisen. Das heißt, vor Beginn und/oder während der Blutbehandlung kann der Volumenstrom des durch den Anionenaustauscher strömenden Blutplasmas bzw. die Förderrate der Plasmapumpe für das Fördern einer vorwählbaren Menge an Blutplasma durch einen Bediener, wie beispielsweise einen Arzt oder Krankenhauspersonal, an der Vorrichtung zur extrakorporalen Blutbehandlung eingestellt und je nach gewünschten Therapiebedingungen angepasst werden. Dadurch kann der zeitliche Verlauf der extrakorporalen Blutbehandlung und die Durchflussrate durch den Anionenaustauscher flexibel verändert werden. Alternativ oder zusätzlich kann die Förderrate der Blutpumpe an der Vorrichtung eingestellt und angepasst werden.

Wie bereits vorstehend beschrieben, wird neben dem LPS auch Heparin durch dessen negative Gesamtladung an dem Anionenaustauscher adsorbiert. Jedoch können darüber hinaus auch andere Plasmaproteine oder sich in dem Plasma befindliche Medikamente bzw. Wirkstoffe mit einer negativen Gesamtladung an den Anionenaustauscher binden und damit aus dem Blutplasma entfernt werden. Um ein ungewolltes Entfernen weiterer Plasmaproteine und/oder Wirkstoffe auszugleichen, kann die vorstehend beschriebene Antikoagulationssteuerungsvorrichtung ein Mittel zum Nachführen von Medikamenten und/oder bluteigener Substanzen, die von dem Anionenaustauscher adsorbiert werden, in den dritten Leitungsabschnitt aufweisen.

Gemäß der Offenbarung kann vorzugsweise die Antikoagulationssteuervorrichtung derart konzipiert sein, dass die Blutpumpe in dem ersten Leitungsabschnitt stromaufwärts der Pumpe, insbesondere der Heparinpumpe, und des Plasmaseparators angeordnet ist. Alternativ ist es jedoch auch möglich, die Blutpumpe an einer anderen Position innerhalb des extrakorporalen Blutkreislaufs anzuordnen. Des Weiteren ist die Plasmapumpe in dem zweiten Leitungsabschnitt stromabwärts des Plasmaseparators, aber stromaufwärts des Anionenaustauschers angeordnet. Auch hier ist eine alternative Anordnung der Plasmapumpe an einer anderen Position innerhalb des zweiten Leitungsabschnitts möglich.

Offenbarungsgemäß kann der Anionenaustauscher eine Diethylaminoethylcellulose (DEAE)-modifizierte Oberfläche aufweisen. Dies ist insofern von Vorteil, als dass vergleichbare Materialen oder Beschichtungen des Anionenaustauschers, wie beispielsweise Polymyxin B (PMB), LPS in der Praxis (Falkenhagen et al., Int J Artif Organs 2014; 37 (3): 222-232) wesentlich schlechter aus Blutserum entfernen. Ein Anionenaustauscher mit einer DEAE-modifizierten Oberfläche stellt somit das am besten geeignete Material und die effizienteste Lösung für die Adsorption von LPS aus dem Blut dar.

Kurzbeschreibung der Erfindung

Die vorliegende Offenbarung wird im Folgenden anhand eines bevorzugten Ausführungsbeispiels unter Bezugnahme der beigefügten Zeichnungen näher beschrieben. Es zeigen:

Fig. 1 eine Darstellung zur Veranschaulichung eines Systemaufbaus gemäß der vorliegenden Offenbarung;

Fig. 2 ein Diagramm zur Veranschaulichung der Abnahme der Heparinkonzentration im Blut während einer simulierten Behandlung ohne die Substitution von Heparin; und

Fig. 3 ein Diagramm zur Veranschaulichung der Heparinkonzentration im Blut während einer simulierten Behandlung mit Substitution von Heparin gemäß der vorliegenden Offenbarung.

Beschreibung des Ausführungsbeispiels

Nachstehend wird das Ausführungsbeispiel der vorliegenden Offenbarung auf der Basis der zugehörigen Figuren beschrieben. Gleiche oder funktional äquivalente Merkmale sind in den einzelnen Figuren mit denselben Bezugszeichen versehen.

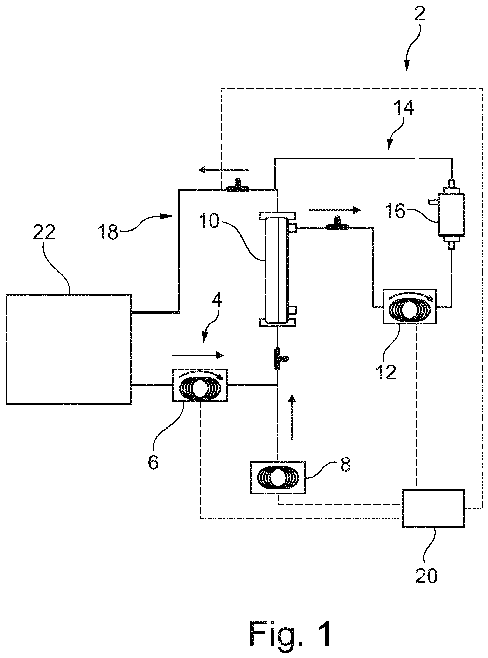

Fig. 1 zeigt einen Systemaufbau einer Antikoagulationssteuerungsvorrichtung 2 für die Simulation eines Verfahrens zur extrakorporalen Blutbehandlung. Dabei weist die Antikoagulationssteuerungsvorrichtung 2 einen ersten Leitungsabschnitt 4 auf, über welchen zu behandelndes Blut, insbesondere eines sich der Blutbehandlung unterziehenden Patienten 22, mittels einer, z.B. peristaltischen, Blutpumpe 6 angesaugt wird. Stromabwärts der Blutpumpe 6 wird dem sich in dem ersten Leitungsabschnitt 4 befindlichen Blut mittels einer Pumpe 8, insbesondere einer Heparinpumpe 8, Heparin zugeführt, um zu gewährleisten, dass das Blut nicht gerinnt. Das mit Heparin versetzte Blut wird schließlich zu einem Plasmaseparator 10 gefördert, in welchem das Blut in seine korpuskulären Bestandteile, d.h. zellulären Bestandteilen wie Erythrozyten oder Leukozyten, einerseits und Blutplasma andererseits getrennt wird. Das Blutplasma enthält dabei Ionen und Proteine, beispielsweise zur Blutgerinnung. Während die korpuskulären Bestandteile des Blutes mit einem Teil des Blutplasmas in dem Plasmaseparator 10 verbleiben, wird ein anderer Teil des Blutplasmas über einen von einer Plasmapumpe 12 erzeugten Unterdruck in einen zweiten Leitungsabschnitt 14 angesaugt und in diesem über einen Anionenaustauscher 16 gefördert.

Durch das Durchströmen des Anionenaustauschers 16 werden Proteine mit negativer Gesamtladung an der Oberfläche des Anionenaustauschers 16 gebunden/adsorbiert und dadurch aus dem Blutplasma entfernt. Eine Beschichtung der Oberfläche des Anionenaustauschers 16 mit DEAE adsorbiert dabei vor allem LPS und Heparin sehr effektiv. Aufgrund der Funktionsweise des Anionenaustauschers 16 werden neben LPS und Heparin jedoch auch Medikamente und andere Plasmaproteine mit negativer Gesamtladung, wie beispielsweise Gerinnungsfaktoren, entfernt. Nach Durchströmen des Anionenaustauschers 16 wird das behandelte Blutplasma in einen stromabwärts des Plasmaseparators 10 und stromabwärts des Anionenaustauschers 16 befindlichen dritten Leitungsabschnitt 18 geführt, in welchem es wieder mit den in dem Plasmaseparator 10 verbliebenen korpuskulären Bestandteilen und dem einen, nicht behandelten Teil des Blutplasmas zusammengeführt und schließlich über den dritten Leitungsabschnitt 18 zurück zu dem Patienten 22 geführt wird.

Damit das nach dem Durchströmen des Anionenaustauschers 16 zusammengeführte Blut aufgrund der Adsorption von Heparin nicht gerinnt und dadurch womöglich einen Abbruch der Blutbehandlung bedingt, ist die Antikoagulationssteuerungsvorrichtung 2 ferner mit einer Steuereinheit 20 vorgesehen, welche das Zuführen von Heparin in dem ersten Leitungsabschnitt 4 mittels der Heparinpumpe 8 derart steuert, dass die Heparinkonzentration in dem dritten Leitungsabschnitt 18, die letztlich zu dem Patienten 22 fließen soll, einen vorbestimmten, vorzugsweise konstanten, Wert annimmt. Dazu erfasst die Steuereinheit 20 einen an der Blutpumpe 6 bestimmten ersten Volumenstrom des Blutplasmaanteils in dem ersten Leitungsabschnitt 4 und einen an der Plasmapumpe 12 bestimmten zweiten Volumenstrom des Blutplasmas in dem zweiten Leitungsabschnitt 14 und setzt diese in Relation zueinander. Um den zweiten Volumenstrom zu bestimmen, kann die Antikoagulationssteuerungsvorrichtung 2 beispielsweise mit in Fig. 1 nicht dargestellten Sensoren versehen sein, die den Volumenstrom an der Plasmapumpe 12 erfassen, oder diesen über die Förderrate der Plasmapumpe 12 ableiten. Auf die gleiche Art und Weise können parallel zu Heparin auch weitere biologisch und/oder pharmakologisch aktive Substanzen mit negativer Gesamtladung zugeführt und gesteuert werden.

Zur Bestimmung des Volumenstroms des Blutplasmaanteils in dem ersten Leitungsabschnitt 4 ist neben der Erfassung des Volumenstroms des Blutes an der Blutpumpe 6 auch der Hämatokrit-Wert notwendig. Obwohl der Hämatokrit-Wert patientenabhängig ist, schwankt er im Verlauf der extrakorporalen Blutbehandlung eines Patienten nicht stark, sodass er im Normalfall als konstant angenommen werden kann. Zur Bestimmung des Volumenstroms des Blutes kann die Antikoagulationssteuerungsvorrichtung 2 wie im Falle der Plasmapumpe 12 Sensoren aufweisen oder diesen anhand der Förderrate der Blutpumpe 6 ableiten. Weiterhin kann die Antikoagulationssteuerungsvorrichtung 2 ein Mittel, beispielsweise in Form einer Benutzeroberfläche, zum Eingeben des Hämatokrit-Werts des Blutes in dem ersten Leitungsabschnitt 4 aufweisen.

Darüber hinaus kann die Antikoagulationssteuerungsvorrichtung 2 analog zum Stand der Technik auch eine in dem dritten Leitungsabschnitt 18 angeordnete Dialyseeinheit und/oder eine Ultrafiltrationseinheit aufweisen.

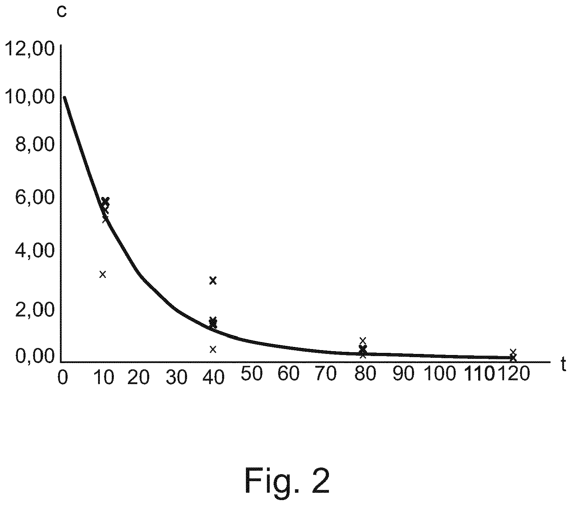

Fig. 2 zeigt ein Diagramm, in welchem die Abnahme der Heparinkonzentration im Blut eines Patienten 22 während einer simulierten Behandlung ohne die Substitution von Heparin veranschaulicht ist. Dabei ist die Heparinkonzentration c in sogenannten International Units pro Milliliter [IU/ml] als Funktion von der Behandlungszeit t in Minuten [min] dargestellt. Die Heparinausgangskonzentration wurde vor der Behandlung, d.h. zum Zeitpunkt 0, bei unterschiedlichen Förderraten der Blutpumpe 6 und Plasmapumpe 12 auf 5 bis 11 IU/ml eingestellt, weshalb pro Messzeitpunkt vier Messwerte aufgezeichnet sind. Anhand von Fig. 2 wird deutlich, dass die Heparinkonzentration im Blut mit fortschreitender Behandlung exponentiell abnimmt.

Das heißt, die Heparinkonzentration c im Blut sinkt ausgehend von der Heparinausgangskonzentration G in Abhängigkeit von der Behandlungszeit t und einer Entfernungskonstante k. Dieser Zusammenhang kann wie folgt dargestellt werden:

$$(1) \qquad \Delta c = k * (G - f(t))\Delta t$$

Stellt man nun Formel (1) nach der Entfernungskonstante k um und integriert nach der Behandlungszeit t ergibt sich folgender Zusammenhang:

$$\Delta c = k * (G - f(t))\Delta t$$

$$\frac{\Delta c}{\Delta t} = k * (G - f(t))$$

$$\frac{\frac{\Delta c}{\Delta t}}{f(t)} = k \qquad G = 0$$

$$ln|f(t)| = -k * t + b$$

$$f(t) = a * e^{-k*t}$$

$$(2) \qquad c_{eff} = c_0 * e^{(-VSplasma/GVplasma)*t}$$

wobei $c_{eff}$ für die effektive Heparinkonzentration zum Zeitpunkt t steht, $c_0$ für die Heparinausgangskonzentration im Blut, VSplasma für den Volumenstrom des Blutplasmas in dem zweiten Leitungsabschnitt 14 an der Plasmapumpe 12 und GVplasma für das Gesamtvolumen des Blutplasmaanteils in dem ersten Leitungsabschnitt 4, insbesondere an der Blutpumpe 6, vor der Zuführung des Heparins durch die Heparinpumpe 8. Aus Formel (2) ergibt sich generell der Zusammenhang, dass für eine Konstanz der Heparinkonzentration in dem dritten Leitungsabschnitt 18, die über die Heparinpumpe 8 zuzuführende Heparinmenge so erhöht werden muss, dass der Heparinteilstrom über den Anionenaustauscher 16 in dem zweiten Leitungsabschnitt 14 kompensiert werden wird, wobei dieser mit dem Plasmafluss, welcher über die Plasmapumpe eingestellt werden kann, korreliert.

In der klinischen Praxis wird jedoch keine Heparinkonzentration verschrieben, sondern eine Heparininfusion mit einem definierten Volumenstrom. Ein gewünschter Heparinvolumenstrom HEP2 in dem dritten Leitungsabschnitt 18, der letztlich zu dem Patienten 22 fließen soll, kann gemäß der vorliegenden Offenbarung vorab der Behandlung eingestellt werden, sodass sich unter Einbeziehung dieses Parameters auf Grundlage von Formel (2) folgender Zusammenhang ergibt:

$$(3) \qquad HEP2 = PF/(BF * (1 - HK/100)) * HEP1$$

Dabei steht PF für den Plasmafluss in dem zweiten Leitungsabschnitt 14, BF für den Blutfluss in dem ersten Leitungsabschnitt 4, HK für den vorher bestimmten Hämatokrit-Wert und HEP1 für die über die Heparinpumpe 8 zuzuführende Heparinmenge, die mit Hilfe der Steuereinheit 20 gesteuert wird.

Der Plasmafluss in dem zweiten Leitungsabschnitt 14 PF lässt sich wie bereits vorstehend erwähnt durch die Drehzahl der Plasmapumpe 12 oder beispielsweise Sensoren erfassen, ebenso wie der Blutfluss in dem ersten Leitungsabschnitt 4 BF, der sich aus der bestimmten Förderrate des Blutes in dem ersten Leitungsabschnitt 4 an der Blutpumpe 6 ergibt. Umgestellt nach HEP1 ergibt sich für Formel (3) nachfolgend:

$$(4) \qquad HEP1 = HEP2/(1 - (PF/(BF * (1 - HK/100))))$$

Um letztlich die zuzuführende Heparinmenge in den ersten Leitungsabschnitt 4 bzw. die Förderrate der Heparinpumpe 8 anhand von Formel (4) zu berechnen, gibt ein Bediener der Antikoagulationssteuerungsvorrichtung 2 beispielsweise über die Benutzeroberfläche HEP2, PF, BF und den bestimmten HK des Patienten 22 vorab oder auch während der Behandlung ein. Die Antikoagulationssteuerungsvorrichtung 2 kann dann anhand der Behandlungsdaten die zuzuführende Heparinmenge in den ersten Leitungsabschnitt 4 bzw. die Förderrate der Heparinpumpe 8 automatisch steuern.

Um HEP2 auch beim Einwirken möglicher Störeinflüsse konstant zu halten, kann die Steuereinheit 20 der Antikoagulationssteuervorrichtung 2 auch als Regeleinheit konzipiert sein. Die Regeleinheit regelt HEP2 dann auf der Grundlage eines in dem dritten Leitungsabschnitt 18 bestimmten Istwerts, dem Verhältnis des Volumenstroms des Blutplasmaanteils in dem ersten Leitungsabschnitt 4 vor der Zuführung des Heparins und dem Volumenstrom des Blutplasmas in dem zweiten Leitungsabschnitt 14 an der Plasmapumpe 12.

So liegt eine Gedanke der vorliegenden Offenbarung darin, dass wenn dem aus dem Plasmaseparator 10 mittels der Plasmapumpe 12 abgezogenen Permeat, d.h. dem (Teil-)Blutplasma-Volumenstrom im zweiten Leitungsabschnitt 14, im Anionenaustauscher 16 die hochtoxischen LPS nur unter gleichzeitiger Mitabtrennung des gleichsam negativ-gesamtgeladenen Heparin entziehen kann, diese Verlustmenge an Heparin im Sinne einer für einen beispielsweise Sepsis-Patienten 22 benötigten konstanten Soll-Konzentration zu substituieren ist. Insofern es sich um eine mitunter lebensnotwendig entgiftende Maßnahme handeln kann, kann es technisch ohne weiteres hingenommen werden, dass ein erster Anteil an LPS nicht ohne einen zweiten Anteil an Heparin dem Blutplasma entzogen werden kann. Damit geht einher, dass der Anionenaustauscher 16 sich deutlich schneller kapazitativ erschöpfen kann, als im Idealfall einer verfügbaren spezifisch für den ersten Anteil an LPS selektiven Trennmatrix der Fall wäre. Mit anderen Worten, wird das absolute Ziel einer lebensrettenden Entgiftung erreicht, indem man einen Überschuss an Matrix des Anionenaustauschers 16 gewissermaßen «unökonomisch» einsetzen kann.

Weiter vorteilhaft dient die Substitution über die im ersten Leitungsabschnitt mittels der Heparinpumpe 8 zuzuführende Heparinmenge der Vermeidung von Koagulation über alle drei Leitungsabschnitte hinweg. Auch in diesem Punkt zeigt die vorliegende Offenbarung einen weiteren Paradigmenwechsel, indem sie von der einleitend beschriebenen Lehre der Fachwelt, einen Anionenaustauscher nach Möglichkeit nicht oder höchstens geringfügig mit Heparin zu beaufschlagen, abweicht.

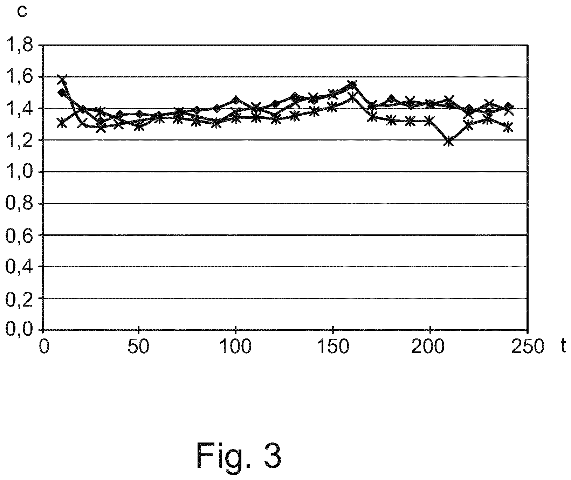

Im Gegensatz zu Fig. 2 zeigt Fig. 3 ein Diagramm zur Veranschaulichung der Heparinkonzentration im Blut eines Patienten 22 während einer simulierten Behandlung mit Substitution von Heparin gemäß der vorliegenden Offenbarung. Auch in Fig. 3 ist die Heparinkonzentration c in sogenannten International Units pro Milliliter [IU/ml] als Funktion von der Behandlungszeit t in Minuten [min] dargestellt. In diesem Fall wurde vorab eine Heparinkonzentration von 1,4 IU/ml eingestellt, die schließlich in dem dritten Leitungsabschnitt 18 vorliegen und zu dem Patienten 22 zurückfließen soll. Die Steuereinheit 20 steuert die zuzuführende Heparinmenge in den ersten Leitungsabschnitt 4 bzw. die Förderrate der Heparinpumpe 8 in Fig. 3 basierend auf Formel (4). Auf diese Weise kann HEP2 über die gesamte Behandlungsdauer hinweg konstant gehalten werden.

Somit ermöglicht die vorliegende Offenbarung den Einsatz von Anionenaustauschern 16 bei gleichzeitigem Einsatz von Heparin im Rahmen einer extrakorporalen Therapie. Damit kann das nach Falkenhagen et al. (Int J Artif Organs 2014; 37 (3): 222-232) am besten geeignete Material für die Entfernung von Endotoxinen aus dem Blut zur Anwendung in einer extrakorporalen Therapie am Menschen bei Sepsis gebracht werden.

Bezugszeichenliste

| 2 | Antikoagulationssteuerungsvorrichtung |
|---|---|
| 4 | Erster Leitungsabschnitt |
| 6 | Blutpumpe |
| 8 | Pumpe/Heparinpumpe |
| 10 | Plasmaseparator |
| 12 | Plasmapumpe |
| 14 | Zweiter Leitungsabschnitt |
| 16 | Anionenaustauscher |
| 18 | Dritter Leitungsabschnitt |
| 20 | Steuereinheit |
| 22 | Patient |

**Patentansprüche**

1.  Antikoagulationssteuerungsvorrichtung (2) zur Anwendung während einer extrakorporalen Blutbehandlung mit:

    einem Mittel, insbesondere einer Blutpumpe (6), zum Fördern von Blut in einem ersten Leitungsabschnitt (4);
    einem Mittel, insbesondere einer Pumpe (8), zum Zuführen einer Anzahl von biologisch und/oder pharmakologisch aktiven Substanzen mit negativer Gesamtladung, insbesondere von Heparin, zu dem Blut in den ersten Leitungsabschnitt (4);
    einem Mittel, insbesondere einem Plasmaseparator (10), zum Separieren des mit der Anzahl von biologisch und/oder pharmakologisch aktiven Substanzen mit negativer Gesamtladung versetzten Blutes in korpuskuläre Blutbestandteile und Blutplasma;
    einem Mittel, insbesondere einer Plasmapumpe (12), zum Fördern des separierten Blutplasmas in einem zweiten Leitungsabschnitt (14) über einen Anionenaustauscher (16) zur Adsorption von Lipopolysacchariden;
    einem Mittel zum Zusammenführen des behandelten Blutplasmas und der korpuskulären Blutbestandteile in einem dritten Leitungsabschnitt (18), und
    einem Mittel zum Bestimmen eines ersten Volumenstroms des Blutplasmaanteils in dem ersten Leitungsabschnitt (4) vor der Zuführung der Anzahl von biologisch und/oder pharmakologisch aktiven Substanzen mit negativer Gesamtladung, insbesondere an der Blutpumpe (6); und
    einem Mittel zum Bestimmen eines zweiten Volumenstroms des Blutplasmas in dem zweiten Leitungsabschnitt (14) stromaufwärts oder stromabwärts des Anionenaustauschers (16), insbesondere an der Plasmapumpe (12);
    **gekennzeichnet durch**
    eine Steuereinheit (20), die dazu ausgelegt ist, eine dem Blut vor dem Separieren zugeführte Menge der Anzahl von biologisch und/oder pharmakologisch aktiven Substanzen mit negativer Gesamtladung auf der Grundlage des Verhältnisses von dem ersten Volumenstrom und dem zweiten Volumenstrom derart zu steuern, dass eine Konzentration der Anzahl von biologisch und/oder pharmakologisch aktiven Substanzen mit negativer Gesamtladung in dem dritten Leitungsabschnitt (18) nach dem Zusammenführen des behandelten Blutplasmas und der korpuskulären Blutbestandteile einen vorbestimmten, insbesondere konstanten, Sollwert annimmt.

2.  Antikoagulationssteuerungsvorrichtung (2) nach Anspruch 1, **gekennzeichnet durch**:

    ein Mittel zum Bestimmen eines Istwerts der Konzentration der Anzahl von biologisch und/oder pharmakologisch aktiven Substanzen mit negativer Gesamtladung in dem dritten Leitungsabschnitt (18); und
    ein Mittel zum Regeln der Konzentration der Anzahl von biologisch und/oder pharmakologisch aktiven Substanzen mit negativer Gesamtladung in dem dritten Leitungsabschnitt (18) auf der Grundlage des bestimmten Istwerts, dem Verhältnis von dem ersten Volumenstrom und dem zweiten Volumenstrom und der zugeführten Menge der Anzahl von biologisch und/oder pharmakologisch aktiven Substanzen mit negativer Gesamtladung im ersten Leitungsabschnitt (4).

3.  Antikoagulationssteuerungsvorrichtung (2) nach Anspruch 1 oder 2, **gekennzeichnet durch**:

    ein Mittel zum Eingeben eines Hämatokrit-Werts des Blutes in dem ersten Leitungsabschnitt (4); und

ein Mittel zum Bestimmen des Volumenstroms des Blutplasmaanteils in dem ersten Leitungsabschnitt (4) auf der Grundlage der Förderrate der Blutpumpe (6) und des eingegebenen Hämatokrit-Werts.

4. Antikoagulationssteuerungsvorrichtung (2) nach einem der Ansprüche 1 bis 3, **gekennzeichnet durch**:

ein Mittel zum Bestimmen eines Hämatokrit-Werts des Blutes in dem ersten Leitungsabschnitt (4);
ein Mittel zum Bestimmen eines Volumenstroms des Blutes in dem ersten Leitungsabschnitt (4), insbesondere Bestimmen einer Förderrate der Blutpumpe (6); und
ein Mittel zum Bestimmen des Volumenstroms des Blutplasmaanteils in dem ersten Leitungsabschnitt (4) auf der Grundlage des bestimmten Volumenstroms des Blutes in dem ersten Leitungsabschnitt (4) und des bestimmten Hämatokrit-Werts.

5. Antikoagulationssteuerungsvorrichtung (2) nach einem der Ansprüche 1 bis 4, **gekennzeichnet durch**:
ein Mittel zum Einstellen des zweiten Volumenstroms des durch den Anionenaustauscher (16) strömenden Blutplasmas, insbesondere die Plasmapumpe (12).

6. Antikoagulationssteuerungsvorrichtung (2) nach einem der Ansprüche 1 bis 5, **gekennzeichnet durch**:
ein Mittel zum Nachführen von Medikamenten und/oder bluteigener Substanzen, die von dem Anionenaustauscher (16) adsorbiert werden, in den dritten Leitungsabschnitt (18).

7. Antikoagulationssteuerungsvorrichtung (2) nach einem der Ansprüche 1 bis 6, **dadurch gekennzeichnet, dass** die Blutpumpe (6) in dem ersten Leitungsabschnitt (4) stromaufwärts der Pumpe (8) und des Plasmaseparators (10) angeordnet ist.

8. Antikoagulationssteuerungsvorrichtung (2) nach einem der Ansprüche 1 bis 7, **dadurch gekennzeichnet, dass** die Plasmapumpe in dem zweiten Leitungsabschnitt (14) stromabwärts des Plasmaseparators (10), aber stromaufwärts des Anionenaustauschers (16) angeordnet ist.

9. Antikoagulationssteuerungsvorrichtung (2) nach einem der Ansprüche 1 bis 8, **dadurch gekennzeichnet, dass** der Anionenaustauscher (16) eine Diethylaminoethylcellulose (DEAE)-modifizierte Oberfläche aufweist.

**Claims**

1. An anticoagulation control device (2) for application during extracorporeal blood treatment, said anticoagulation control device (2) comprising:

a means, in particular a blood pump (6), for conveying blood in a first line section (4);
a means, in particular a pump (8), for supplying a number of biologically and/or pharmacologically active substances of negative total charge, in particular heparin, to the blood in the first line section (4);
a means, in particular a plasma separator (10), for separating blood added with the number of biologically and/or pharmacologically active substances of negative total charge into corpuscular blood components and blood plasma;
a means, in particular a plasma pump (12), for conveying the separated blood plasma in a second line section (14) via an anion exchanger (16) for adsorption of lipopolysaccharides;
a means for bringing the treated blood plasma and the corpuscular blood components together in a third line section (18), and
a means for determining a first volumetric flow rate of the blood plasma fraction in the first line section (4) before the supplying of the number of biologically and/or pharmacologically active substances of negative total charge, in particular at the blood pump (6); and
a means for determining a second volumetric flow rate of the blood plasma in the second line section (14) upstream or downstream of the anion exchanger (16), in particular at the plasma pump (12);
**characterized by**
a control unit (20) which is adapted to control a quantity of the number of biologically and/or pharmacologically active substances of negative total charge, wherein said substances are supplied to the blood before the separating, on the basis of the ratio of the first volumetric flow rate and the second volumetric flow rate, in such a way that, after the treated blood plasma and the corpuscular blood components have been brought together, a concentration of the number of biologically and/or pharmacologically active substances of negative total charge in the third line section (18) obtains a predetermined, in particular constant, target value.

2. The anticoagulation control device (2) according to claim 1, **characterized by**:

a means for determining an actual value of the concentration of the number of biologically and/or pharmacologically active substances of negative total charge in the third line section (18); and
a means for controlling the concentration of the number of biologically and/or pharmacologically active substances of negative total charge in the third line section (18) on the basis of the determined actual value, the ratio of the first volumetric flow rate and the second volumetric flow rate, and the supplied quantity of the number of biologically and/or pharmacologically active substances of negative total charge in the first line section (4).

3. The anticoagulation control device (2) according to claim 1 or 2, **characterized by**:

a means for inputting a hematocrit value of the blood in the first line section (4); and
a means for determining the volumetric flow rate of the blood plasma fraction in the first line section (4) on the basis of the conveying rate of the blood pump (6) and the input hematocrit value.

4. The anticoagulation control device (2) according to any of claims 1 to 3, **characterized by**:

a means for determining a hematocrit value of the blood in the first line section (4);
a means for determining a volumetric flow rate of the blood in the first line section (4), in particular for determining a conveying rate of the blood pump (6); and
a means for determining the volumetric flow rate of the blood plasma fraction in the first line section (4) on the basis of the determined volumetric flow rate of the blood in the first line section (4) and the determined hematocrit value.

5. The anticoagulation control device (2) according to any of claims 1 to 4, **characterized by**:
a means for setting the second volumetric flow rate of the blood plasma flowing through the anion exchanger (16), in particular the plasma pump (12).

6. The anticoagulation control device (2) according to any of claims 1 to 5, **characterized by**:
a means for additionally supplying medicines and/or blood-inherent substances, which are adsorbed by the anion exchanger (16), into the third line section (18).

7. The anticoagulation control device (2) according to any of claims 1 to 6, **characterized in that** the blood pump (6) is arranged in the first line section (4) upstream of the pump (8) and of the plasma separator (10).

8. The anticoagulation control device (2) according to any of claims 1 to 7, **characterized in that** the plasma pump is arranged in the second line section (14) downstream of the plasma separator (10), but upstream of the anion exchanger (16).

9. The anticoagulation control device (2) according to any of claims 1 to 8, **characterized in that** the anion exchanger (16) has a surface modified with diethylaminoethyl cellulose (DEAE).

**Revendications**

1. Dispositif de commande d'anticoagulation (2) à utiliser pendant un traitement extracorporel du sang avec :

un moyen, en particulier une pompe d'assistance circulatoire (6), pour refouler du sang dans une première partie de conduite (4) ;
un moyen, en particulier une pompe (8), pour amener un nombre de substances biologiquement et/ou pharmaceutiquement actives avec une charge totale négative, en particulier d'héparine, au sang dans la première partie de conduite (4) ;
un moyen, en particulier un séparateur de plasma (10), pour séparer le sang mélangé au nombre de substances biologiquement et/ou pharmaceutiquement actives avec une charge totale négative en fractions sanguines corpusculaires et plasma sanguin ;
un moyen, en particulier une pompe à plasma (12), pour refouler le plasma sanguin séparé dans une deuxième partie de conduite (14) par l'intermédiaire d'un échangeur d'anions (16) pour l'adsorption de lipopolysaccharides ;

un moyen pour réunir le plasma sanguin traité et les fractions sanguines corpusculaires dans une troisième partie de conduite (18), et

un moyen pour déterminer un premier débit volumétrique de la part de plasma sanguin dans la première partie de conduite (4) avant l'amenée du nombre de substances biologiquement et/ou pharmaceutiquement actives avec une charge totale négative, en particulier sur la pompe d'assistance circulatoire (6) ; et

un moyen pour déterminer un deuxième débit volumétrique du plasma sanguin dans la deuxième partie de conduite (14) en amont ou en aval de l'échangeur d'anions (16), en particulier sur la pompe à plasma (12) ;

**caractérisé par**

une unité de commande (20), qui est conçue pour commander une quantité du nombre de substances biologiquement et/ou pharmaceutiquement actives avec une charge totale négative amenée au sang avant la séparation sur la base du rapport du premier débit volumétrique et du deuxième débit volumétrique, de telle sorte qu'une concentration du nombre de substances biologiquement et/ou pharmaceutiquement actives avec une charge totale négative dans la troisième partie de conduite (18) après la réunion du plasma sanguin traité et des fractions sanguines corpusculaires prend une valeur prédéfinie, en particulier constante.

2. Dispositif de commande d'anticoagulation (2) selon la revendication 1, **caractérisé par** :

un moyen pour déterminer une valeur réelle de la concentration du nombre de substances biologiquement et/ou pharmaceutiquement actives avec une charge totale négative dans la troisième partie de conduite (18) ; et

un moyen pour réguler la concentration du nombre de substances biologiquement et/ou pharmaceutiquement actives avec une charge totale négative dans la troisième partie de conduite (18) sur la base de la valeur réelle déterminée, du rapport du premier débit volumétrique et du deuxième débit volumétrique et de la quantité amenée du nombre de substances biologiquement et/ou pharmaceutiquement actives avec une charge totale négative dans la première partie de conduite (4).

3. Dispositif de commande d'anticoagulation (2) selon la revendication 1 ou 2, **caractérisé par** :

un moyen pour entrer une valeur d'hématocrite du sang dans la première partie de conduite (4) ; et

un moyen pour déterminer le débit volumétrique de la part de plasma sanguin dans la première partie de conduite (4) sur la base de la vitesse de refoulement de la pompe d'assistance circulatoire (6) et de la valeur d'hématocrite entrée.

4. Dispositif de commande d'anticoagulation (2) selon l'une quelconque des revendications 1 à 3, **caractérisé par** :

un moyen pour déterminer une valeur d'hématocrite du sang dans la première partie de conduite (4) ;

un moyen pour déterminer un débit volumétrique du sang dans la première partie de conduite (4), en particulier déterminer une vitesse de refoulement de la pompe d'assistance circulatoire (6) ; et

un moyen pour déterminer le débit volumétrique de la part de plasma sanguin dans la première partie de conduite (4) sur la base du débit volumétrique déterminé du sang dans la première partie de conduite (4) et de la valeur d'hématocrite déterminée.

5. Dispositif de commande d'anticoagulation (2) selon l'une quelconque des revendications 1 à 4, **caractérisé par** : un moyen pour régler le deuxième débit volumétrique du plasma sanguin s'écoulant à travers l'échangeur d'anions (16), en particulier la pompe à plasma (12).

6. Dispositif de commande d'anticoagulation (2) selon l'une quelconque des revendications 1 à 5, **caractérisé par** : un moyen pour faire suivre des médicaments et/ou substances propres au sang, qui sont adsorbés par l'échangeur d'anions (16), dans la troisième partie de conduite (18).

7. Dispositif de commande d'anticoagulation (2) selon l'une quelconque des revendications 1 à 6, **caractérisé en ce que** la pompe d'assistance circulatoire (6) est disposée dans la première partie de conduite (4) en amont de la pompe (8) et du séparateur de plasma (10).

8. Dispositif de commande d'anticoagulation (2) selon l'une quelconque des revendications 1 à 7, **caractérisé en ce que** la pompe d'assistance circulatoire est disposée dans la deuxième partie de conduite (14) en aval du séparateur de plasma (10), mais en amont de l'échangeur d'anions (16).

13

EP 3 877 016 B1

9. Dispositif de commande d'anticoagulation (2) selon l'une quelconque des revendications 1 à 8, **caractérisé en ce que** l'échangeur d'anions (16) présente une surface modifiée par la diéthylaminoéthyl cellulose (DEAE).

14

Fig. 1

Fig. 2

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 3135814 A1
- EP 0705845 B1
- US 20160038666 A1
- AT 509192 A4

**In der Beschreibung aufgeführte Nicht-Patentliteratur**

- **FALKENHAGEN et al.** *Int J Artif Organs,* 2014, vol. 37 (3), 222-232